(19) Europäisches Patentamt
European Patent Office
Office européen des brevets

(11) **EP 3 046 604 B1**

(12) **EUROPEAN PATENT SPECIFICATION**

(45) Date of publication and mention
of the grant of the patent:
**06.11.2019 Bulletin 2019/45**

(21) Application number: **14767010.3**

(22) Date of filing: **18.09.2014**

(51) Int Cl.:
*A61M 5/50* *(2006.01)*     *A61M 5/31* *(2006.01)*
*A61M 5/24* *(2006.01)*     *A61M 5/14* *(2006.01)*

(86) International application number:
**PCT/EP2014/069916**

(87) International publication number:
**WO 2015/040122 (26.03.2015 Gazette 2015/12)**

(54) **CONDITIONAL REQUIRED PRIMING**

BEDINGT ERFORDERLICHE GRUNDIERUNG

AMORÇAGE NÉCESSAIRE CONDITIONNEL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.09.2013 EP 13185258**

(43) Date of publication of application:
**27.07.2016 Bulletin 2016/30**

(73) Proprietor: **Sanofi-Aventis Deutschland GmbH
65929 Frankfurt am Main (DE)**

(72) Inventor: **HOLTWICK, Marc
65926 Frankfurt am Main (DE)**

(74) Representative: **Derry, Paul Stefan et al
Venner Shipley LLP
200 Aldersgate
London EC1A 4HD (GB)**

(56) References cited:
**WO-A1-94/12235      WO-A1-2012/160157
WO-A2-2012/126745    US-B1- 6 368 314**

**Description**

The invention relates to an apparatus for ejecting a fluid having a control unit for controlling the apparatus, the control unit being configured to control ejection of a fluid dose from a fluid reservoir through an ejection channel. For example, the apparatus may be a medical device, in particular a medicament injection device.

The medical device can be an injector, for example a hand-held injector, especially a pen-type injector, that is an injector of the kind that provides for administration by injection of medicinal products from one or more multidose cartridges. In particular, the present invention relates to such injectors where a user may set the dose.

The drug agents may be contained in two or more multiple dose reservoirs, containers or packages, each containing independent (single drug compound) or pre-mixed (co-formulated multiple drug compounds) drug agents.

Certain disease states require treatment using one or more different medicaments. Some drug compounds need to be delivered in a specific relationship with each other in order to deliver the optimum therapeutic dose. The present patent application is of particular benefit where combination therapy is desirable, but not possible in a single formulation for reasons such as, but not limited to, stability, compromised therapeutic performance and toxicology.

For example, in some cases it may be beneficial to treat a diabetic with a long acting insulin (also may be referred to as the first or primary medicament) along with a glucagon-like peptide-1 such as GLP-1 or GLP-1 analog (also may be referred to as the second drug or secondary medicament).

Accordingly, there exists a need to provide devices for the delivery of two or more medicaments in a single injection or delivery step that is simple for the user to perform without complicated physical manipulations of the drug delivery device. The proposed drug delivery device provides separate storage containers or cartridge retainers for two or more active drug agents. These active drug agents are then combined and/or delivered to the patient during a single delivery procedure. These active agents may be administered together in a combined dose or alternatively, these active agents may be combined in a sequential manner, one after the other.

The drug delivery device also allows for the opportunity of varying the quantity of the medicaments. For example, one fluid quantity can be varied by changing the properties of the injection device (e.g., setting a user variable dose or changing the device's "fixed" dose). The second medicament quantity can be changed by manufacturing a variety of secondary drug containing packages with each variant containing a different volume and/or concentration of the second active agent.

The drug delivery device may have a single dispense interface. This interface may be configured for fluid communication with a primary reservoir and with a secondary reservoir of medicament containing at least one drug agent. The drug dispense interface can be a type of outlet that allows the two or more medicaments to exit the system and be delivered to the patient.

The combination of compounds from separate reservoirs can be delivered to the body via a double-ended needle assembly. This provides a combination drug injection system that, from a user's perspective, achieves drug delivery in a manner that closely matches the currently available injection devices that use standard needle assemblies. One possible delivery procedure may involve the following steps:

1. Attach a dispense interface to a distal end of the electro-mechanical injection device. The dispense interface comprises a first and a second proximal needle. The first and second needles pierce a first reservoir containing a primary compound and a second reservoir containing a secondary compound, respectively.

2. Attach a dose dispenser, such as a double-ended needle assembly, to a distal end of the dispense interface. In this manner, a proximal end of the needle assembly is in fluidic communication with both the primary compound and secondary compound.

3. Dial up/set a desired dose of the primary compound from the injection device, for example, via a graphical user interface (GUI).

4. After the user sets the dose of the primary compound, the micro-processor controlled control unit may determine or compute a dose of the secondary compound and preferably may determine or compute this second dose based on a previously stored therapeutic dose profile. It is this computed combination of medicaments that will then be injected by the user. The therapeutic dose profile may be user selectable. Alternatively, the user can dial or set a desired dose of the secondary compound.

5. Optionally, after the second dose has been set, the device may be placed in an armed condition. The optional armed condition may be achieved by pressing and/or holding an "OK" or an "Arm" button on a control panel. The armed condition may be provided for a predefined period of time during which the device can be used to dispense the combined dose.

6. Then, the user will insert or apply the distal end of the dose dispenser (e.g. a double ended needle assembly) into the desired injection site. The dose of the combination of the primary compound and the secondary compound (and potentially a third medicament) is administered by activating an injection user interface (e.g. an injection button).

Both medicaments may be delivered via one injection needle or dose dispenser and in one injection step. This offers a convenient benefit to the user in terms of reduced user steps compared to administering two separate injections.

There is a general desire to improve user safety and reliability of apparatuses for ejecting a fluid. User safety and reliability of such an apparatus may however be at risk if the fluid to be ejected degrades or contaminates within the ejection channel, for example due to microorganisms such as bacteria or yeast/fungi appearing or increasing over an acceptable level.

For medical devices it is particularly important to maintain sterility and thereby microbiological integrity of the drugs used with the devices. Medicament injection devices often comprise disposable parts that may be attached to the device and have direct contact with the drug, for example by forming part of the ejection channel. These disposable parts are generally sterilized and sealed so that sterility is maintained until they are attached to the device. To maintain sterility and microbiological integrity of the drug within such a part after the sterile seal of the part is broken, drugs often contain preservatives such as m-cresol. m-cresol causes a decrease of the bacteria or yeast/fungi concentration within, for example, an ejection channel formed by the disposable part.

Medical devices have to fulfil different criteria to be approved for sale or to be accepted by customers, for example the Ph.Eur. (European Pharmacopoeia) criterion A or B or the USP (US Pharmacopeial) criteria. These criteria require a sufficient preservative efficacy so that if a certain amount of bacteria or yeast/fungi occurs in the drugs, their concentrations decrease by a certain magnitude over a defined time.

The Ph. Eur. and USP criteria are given in table 1, wherein the numbers designate the required order of magnitude in CFU (colony forming unit) decrease as a function of time since the time of contamination (T0).

Table 1: Ph.Eur. and USP criteria

| Criterion | Contaminant | T0 | 6h | 24h | 7d | 14d | 28d |
|---|---|---|---|---|---|---|---|
| Ph.Eur. A | Bacteria | - | 2 | 3 | - | - | not detectable |
| | Yeast/Fungi | - | - | - | 2 | - | no increase |
| Ph.Eur. B | Bacteria | - | - | 1 | 3 | - | no increase |
| | Yeast/Fungi | - | - | - | - | 1 | no increase |
| USP | Bacteria | - | - | - | 1.0 | 3.0 | no increase |
| | Yeast/Fungi | - | - | - | no increase | no increase | no increase |

To fulfil these criteria, a sufficient preservative (e.g. m-cresol) concentration is necessary within the drug solution such as for example 2.7 mg/ml m-cresol, which was found to be sufficient to fulfil both, Ph. Eur. criterion A and USP criteria.

Some medical devices are typically used in certain time intervals, such as a medicament delivery device that may be used by a patient every day for medicament administration. During the time between two consecutive daily medicament administrations, i.e. during a time of about 24 hours, there is usually little risk for an ejection channel contamination as the preservative contained in the residual fluid volume within the ejection channel suppresses excessive growth of microorganisms.

However, it may occur that the time length between two ejections is prolonged, for example because the user forgets to use the device or because he is alternately using two different devices. It is desirable that sterility and microbiological integrity is also maintained in such cases, i.e. for longer time length between two ejections from the device.

WO 2012/160157 A1 discloses a medicament delivery device for the administration of one or more drug agents. The device has a priming mode and a drug delivery mode. The device includes a controller operative for controlling the device to perform a priming operation.

WO 2012/126745 A2 discloses an infusion system. A controller selectively effects a priming operation. The infusion system is operable to be primed automatically.

WO 94/12235 A1 discloses a control system for use with an automated intravenous drug and fluid infusion system having plural pumping channels that operate independently for intravenously infusing drugs and fluid. The system functions include preventing priming of a channel unless verification is provided that the channel is not connected to a patient and initiating the priming of each of the pumping channels independently.

US 6 368 314 B1 discloses a process for the monitoring of the pressure of a product fluid to be administered in dosed amounts during an infusion or injection.

In light of the aforementioned, it is inter-alia an object of the present invention to provide an apparatus, in particular a medical device, for ejecting a fluid with improved used safety and reliability, in particular in case of long time length without ejection of a fluid.

According to the present invention there is provided an apparatus according to claim 1 and a system according to claim 14.

The apparatus may be a delivery device, especially a drug delivery device such as a medical device configured to eject a drug agent (e.g. a dose of a medicament) such as an infusion device or an injection device, for instance an insulin injection pen. Injection devices may be used either by medical personnel or by patients themselves. As an example, type-1 and type-2 diabetes may be treated with patients themselves by injection of insulin doses for example once or several times per day.

In particular, the apparatus may be a medical device configured to deliver (i.e. eject) at least two drug agents from separate cartridges situated in two separate retainers.

Alternatively, the apparatus may for instance be configured to deliver (i.e. eject) a two-component adhesive from separate cartridges comprising a first component of the two-component adhesive (i.e. a binder) and a second component of the two-component adhesive (i.e. a hardener) respectively.

The apparatus comprises a control unit for controlling the apparatus. The control unit is preferably a micro-processor control unit comprising a micro-processor and a storage such as a RAM, a ROM, a flash memory, a hard disk or the like containing commands the execution of which by the micro-processor causes control of the apparatus.

The control unit is configured to control fluid dose ejection steps, during which a fluid dose is ejected from a fluid reservoir through an ejection channel. A fluid dose ejection step is understood to mean that a defined dose of a fluid is expelled from the fluid reservoir to be ejected out of the apparatus. In case of a medicament delivery device, such a dose could for example be a medicament bolus to be administered to a patient. The fluid reservoir may be an integral part of the apparatus. The apparatus may also provide a connector or a retainer for a cartridge containing the fluid. The ejection channel is understood to designate a channel for fluid communication between the fluid reservoir and an outlet of the apparatus, through which the fluid dose is ejected from the apparatus. In case of a medicament injection device, the ejection channel may for example be a channel between a medicament containing cartridge and an outlet of a needle assembly connected to the device for administering the drug to a patient.

The control unit is configured to determine a priming time length, the priming time length being the length of time since the last ejection of a fluid volume through the ejection channel. For this purpose, the control unit may for example store the time when a fluid volume is ejected through the ejection channel and subtract this time from the current time, the result of this subtraction yielding the priming time length. For determining the actual time, the control unit may comprise or be connected to a clock. Alternatively, the control unit may also start a time measurement when a fluid volume is ejected through the ejection channel and reset the time measurement when again a volume is ejected through the ejection channel. The current time from the time measurement then yields the current priming time length.

The control unit is configured to perform a priming test step comprising the step of determining whether the priming time length is longer than a predefined priming threshold time length. The predefined priming threshold time length may for example be a value stored in a variable in the storage of the control unit.

The priming test step further comprises the step of setting the apparatus into a status, in which a further fluid dose ejection step is only allowed after execution of an ejection channel priming step if the priming time length is longer than the priming threshold time length.

Setting the apparatus into a particular status may for example comprise setting a status variable in a storage of the control unit to a predefined value, wherein the control unit is configured such that at least one of the possible control steps of the apparatus is a function of the status variable. For example, value "0" in the status variable may indicate regular operation, "1" may indicate a stand-by status and "2" may indicate a status, in which a further fluid dose ejection step is disallowed. Alternatively, setting the apparatus into a particular status may for example comprise jumping to a particular position in the program flow of a computer program run by the control unit, such as a particular subroutine or a particular command sequence.

In a status in which a further fluid dose ejection step is temporarily disallowed, the control unit may still allow ejection of a fluid volume during steps different from fluid dose ejection steps, in particular during an ejection channel priming step.

The ejection channel priming step designates a step during which sterility and microbiological integrity of the ejection channel is ensured or restored.

In an in-use medicament stability study of a medicament delivery device, it was determined that the m-cresol content of the residual fluid volume in a disposable part of the device such as a dispense interface or a needle assembly forming the ejection channel, is affected over time if it is not replaced by a fresh volume of the fluid. If too much time has passed since the last ejection of a fluid volume through the ejection channel, the m-cresol concentration in the residual fluid volume may have dropped below a level that is sufficient to fulfil, e.g., PhEur criterion A or the USP criteria, so that the ejection channel and the fluid therein may have become contaminated by bacteria or yeast/fungi.

To ensure or restore sterility and microbiological integrity of the ejection channel, the ejection channel priming step may in particular comprise the step of ejecting of a fluid through the ejection channel, so that the ejection channel is flushed with fresh fluid from the fluid reservoir. The ejection channel priming step may in addition comprise the step of replacing a part of the ejection channel, such as a disposable part, by a new sterile part before the step of ejecting a fluid through the ejection channel.

With the control unit being configured as described above, an apparatus is provided that prevents fluid dose ejection steps if there is a risk of ejection channel contamination. It was found that for maintaining microbiological integrity of the ejection channel and the fluid therein, the ejection channel has to be flushed by fluid within certain time intervals, so that for example the preservative content such as the m-cresol content is raised back to a sufficient level. By forcing an ejection channel priming step if the last fluid ejection is longer ago than a predefined priming threshold time length, the apparatus ensures that a priming takes place before further fluid dose ejection and therefore prevents contaminated fluid doses to be ejected from the apparatus.

The object described above is furthermore at least in part solved by a system comprising an apparatus as described above and a disposable assembly attached to a connection part of the apparatus, the disposable assembly thereby forming at least part of the ejection channel.

The advantages described above for the apparatus apply accordingly for the system described above.

A number of embodiments of the apparatus and the system will be described in the following. Although these embodiments are described in particular with reference to the apparatus, they are not limited to the apparatus, but also apply accordingly for the system comprising such an apparatus described above.

According to an embodiment of the apparatus, the priming time length is the length of time since the last ejection of a fluid volume through the ejection channel, which fluid volume is equal or larger than a predefined minimum ejection volume. In this way it is ensured that the priming time length is determined from a fluid volume ejection event, during which a sufficient amount of fluid was flushed through the ejection channel, so that microbiological integrity was maintained or restored therein, for example by raising a preservative concentration within the ejection channel to a safe level.

The predefined minimum ejection volume may for example be the volume of the ejection channel. This ensures that the ejection channel is flushed completely with fresh fluid, so that the fluid in the ejection channel may be completely exchanged.

According to a further embodiment of the apparatus the control unit is configured to determine the priming time length by determining the time length since the last performance of a step from a group of steps, the group of steps at least comprising a fluid dose ejection step and/or an ejection channel priming step. Ejection of a fluid volume, in particular of a fluid volume of at least the minimum ejection volume, may occur during particular steps performed by the control unit. Therefore, the control unit may easily determine the time of the last ejection of a fluid volume by determining the time of the last execution of such a step. A fluid volume of sufficient amount, in particular at least equal to the minimum ejection volume, is typically ejected during a fluid dose ejection step, i.e. during a regular ejection of a dose according to the purpose of the device, for example a medicament bolus ejection in case of a medicament delivery device. The time of an ejection channel priming step comprising ejection of a fluid volume, in particular of sufficient size, may also be used as starting point for determining the priming time length.

According to a further embodiment of the apparatus the ejection channel priming step comprises ejection of a fluid volume through the ejection channel, which fluid volume is equal or larger than a predefined minimum priming volume. It was found during experiments that sterility and microbiological integrity within the ejection channel may be restored by flushing the ejection channel with a fluid volume that is sufficient to flush bacteria or yeast/fungi out of the ejection channel and/or increase the preservative content within he ejection channel to a safe level.

The minimum priming volume may for example be equal to or larger than the volume of the ejection channel, in particular from the fluid reservoir to the outlet of the ejection channel. In this way, the fluid within the ejection channel may be completely exchanged. If for example a disposable assembly is attachable to the apparatus, the disposable assembly forming part of the ejection channel, the minimum priming volume may in particular be in particular be equal to or larger than the volume of the ejection channel taking into account the volume of the part of the ejection channel formed by the disposable assembly. If the disposable assembly comprises more than one part, for example a dispense interface and a needle assembly, the control unit may be configured to perform different priming steps, such as a step for only priming one of the parts of the disposable assembly such as the needle assembly and a step for priming all parts of the disposable assembly. In this case, the control unit may in particular be configured such that a priming step for all parts of the disposable assembly is performed during the ejection channel priming step to maintain or restore microbiological integrity of the ejection channel.

The minimum priming volume may also be equal to or larger than the minimum ejection volume.

The control unit may be configured to perform different types of priming steps, for example a needle priming step for priming the needle or a needle assembly.

According to a further embodiment the apparatus comprises a cartridge retainer configured to hold a fluid containing cartridge. The apparatus may also comprise more than one, for example two cartridge retainers for each holding a fluid containing cartridge. In the case of more than one cartridge retainer, the ejection channel priming step may comprise ejection of a fluid volume through the ejection channel from each one of these cartridges. This allows maintaining or restoring the microbiological integrity of the whole ejection channel.

According to a further embodiment the apparatus comprises a connector for removably attaching thereto a disposable assembly, the disposable assembly forming at least part of the ejection channel when attached to the connector. Forming part of the ejection channel is understood to encompass that the disposable assembly forms the complete ejection channel. Sterility and microbiological integrity of the ejection channel may be improved by a using disposable assembly which after a certain time may be exchanged by a new one. However, there may still be a risk of ejection channel contamination, for example if the disposable assembly is used longer than expected or if the time between two uses of the apparatus is longer than the priming threshold time length. Therefore, the embodiment described herein increases user safety of such apparatuses being used with disposable assemblies. The minimum priming volume may in particular be selected such that the volume is equal to or larger than the volume of the ejection channel taking into account the volume of the ejection channel part formed by the disposable assembly.

According to a further embodiment of the apparatus the disposable assembly is a dispense interface. Such dispense interfaces may for example comprise needles to punctuate membranes of fluid containing cartridges situated in cartridge retainers of the apparatus, thereby providing for an ejection channel from the fluid containing cartridges to an outlet of the dispense interface. The dispense interface may comprise a connector for attaching thereto a needle assembly, in particular for administering a medicament to a patient.

According to a further embodiment the apparatus comprises an operating element for operation of the apparatus by a user and the control unit is configured to perform the priming test step once a user operates the operating element. In this way it is ensured that the priming test step is performed each time a user operates a certain operating element of the apparatus. The operating element may be a physical button or a virtual button on a touch screen or a sensor that is activated by a user action.

According to a further embodiment of the apparatus the operating element is an operating element for activating the apparatus, e.g. from a stand-by status. In this way the priming test step is performed each time the apparatus is activated, for example when a user presses any or a particular button of the device. This ensures that the priming test step is performed before the user further operates the apparatus. Alternatively or in addition to that the operating element may be an operating element for preparing or initiating a fluid dose ejection step, for example an injection button. This ensures that a priming test step is carried out before each fluid dose ejection step, so that the microbiological integrity of the ejection channel is ensured just before ejection of a fluid dose. In particular the priming test step may be performed if the user navigates to the dose dial menu to select a dose to be administered.

According to a further embodiment the apparatus comprises a housing with a cap connection part for attaching thereto a cap and a sensor configured to determine whether the cap is attached to the connection part, and the control unit is configured to perform the priming test step upon detachment of the cap. Detachment of the cap is usually a reliable indicator that the user intends to use the apparatus for a fluid dose ejection. Therefore, performing the priming test step upon detachment of the cap ensures that the priming test step is carried out each time when the user is likely to use the apparatus.

In the embodiments described before, the priming test step does not necessarily have to be performed directly after activation of an operating element or detachment of a cap. Rather, the priming test step may also be part of a certain sequence of steps which is carried out on these events. For example, after detaching the cap, the control unit may first perform a self-test of the apparatus before performing the priming test step.

According to a further embodiment of the apparatus, the predefined priming threshold time length is in the range from 24 hours to 10 days, preferably from 36 hours to 5 days. It was found that when using preservatives in the fluid, in particular m-cresol in the case of medicaments, microbiological integrity in the ejection channel is often compromised after a time of one day to ten days, in particular of between five and ten days, if the fluid in the ejection channel is not exchanged. By setting the predefined priming threshold time length accordingly, it is ensured that the ejection channel priming step is performed when the priming time length is larger than the time length that typically results in ejection channel contamination.

According to a further embodiment, the apparatus is a medical device, in particular a medicament injection device. Sterility and microbiological integrity is in particular important for medical devices and medicament injection devices, as contaminated medicaments may cause a severe health risk for the patient. Therefore, the advantages discussed above for the apparatus apply in particular for medical devices.

According to a further embodiment, the apparatus is hand-held. Hand-held apparatuses are often used by laymen such as patients themselves, who often do not have the possibility to externally monitor or maintain the microbiological integrity and sterility of the device. The apparatus described above comprises an integrated system to prevent that contaminated fluid, in particular contaminated medicaments, are ejected from the apparatus and therewith provides improved user safety.

These as well as other advantages of various aspects of the present invention will become apparent to those of ordinary skill in the art by reading the following detailed description, with appropriate reference to the accompanying drawings, in which:

Fig. 1    illustrates a perspective view of a delivery device with an end cap of the device removed;

Fig. 2    illustrates a perspective view of the delivery device distal end showing the cartridge;

Fig. 3    illustrates a perspective view of the delivery device illustrated in Fig. 1 or 2 with one cartridge retainer in an open position;

Fig. 4    illustrates a dispense interface and a dose dispenser that may be removably mounted on a distal end of the delivery device illustrated in Fig. 1;

Fig. 5    illustrates the dispense interface and the dose dispenser illustrated in Fig. 4 mounted on a distal end of the delivery device illustrated in Fig. 1;

Fig. 6    illustrates one arrangement of a needle assembly that may be mounted on a distal end of the delivery device;

Fig. 7    illustrates a perspective view of the dispense interface illustrated in Fig. 4;

Fig. 8    illustrates another perspective view of the dispense interface illustrated in Fig. 4;

Fig. 9    illustrates a cross-sectional view of the dispense interface illustrated in Fig. 4;

Fig. 10    illustrates an exploded view of the dispense interface illustrated in Fig. 4;

Fig. 11    illustrates a cross-sectional view of the dispense interface and needle assembly mounted onto a drug delivery device, such as the device illustrated in Fig. 1;

Fig. 12    illustrates a schematic representation of the control unit of the delivery device illustrated in Fig. 1; and

Fig. 13    shows a flow chart of an exemplary priming test step.

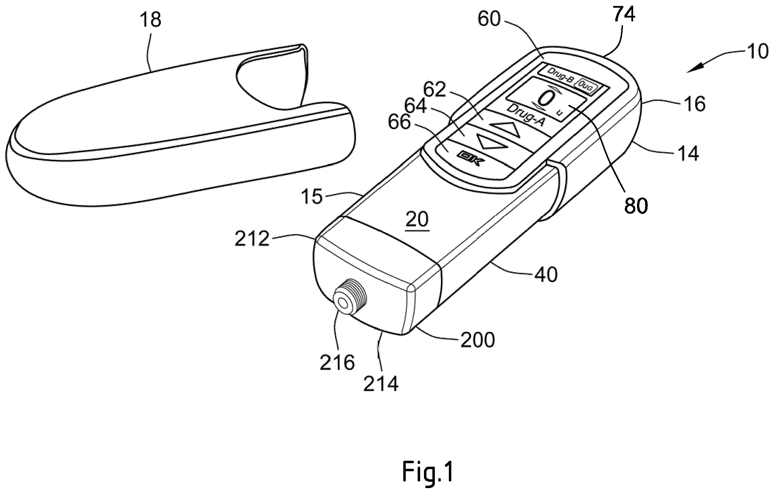

The drug delivery device illustrated in Fig. 1 comprises a main body 14 that extends from a proximal end 16 to a distal end 15. At the distal end 15, a removable end cap or cover 18 is provided. This end cap 18 and the distal end 15 of the main body 14 work together to provide a snap fit or form fit connection so that once the cover 18 is slid onto the distal end 15 of the main body 14, this frictional fit between the cap and the main body outer surface 20 prevents the cover from inadvertently falling off the main body.

The main body 14 contains a micro-processor control unit, an electro-mechanical drive train, and at least two medicament reservoirs. When the end cap or cover 18 is removed from the device 10 (as illustrated in Fig. 1), a dispense interface 200 is mounted to the distal end 15 of the main body 14, and a dose dispenser (e.g., a needle assembly) is attached to the interface. The drug delivery device 10 can be used to administer a computed dose of a second medicament (secondary drug compound) and a variable dose of a first medicament (primary drug compound) through a single needle assembly, such as a double ended needle assembly.

The drive train may exert a pressure on the bung of each cartridge, respectively, in order to expel the doses of the first and second medicaments. For example, a piston rod may push the bung of a cartridge forward a pre-determined amount for a single dose of medicament. When the cartridge is empty, the piston rod is retracted completely inside the main body 14, so that the empty cartridge can be removed and a new cartridge can be inserted.

A control panel region 60 is provided near the proximal end of the main body 14. Preferably, this control panel region 60 comprises a digital display 80 along with a plurality of human interface elements that can be manipulated by a user to set and inject a combined dose. In this arrangement, the control panel region comprises a first dose setting button 62, a second dose setting button 64 and a third button 66 designated with the symbol "OK." In addition, along the most proximal end of the main body, an injection button 74 is also provided (not visible in the perspective view of Fig. 1). The user interface of the drug delivery device may comprise additional buttons, such as a "menu" button, a "back" button, or a "light" button to switch on an illumination of the display.

The cartridge holder 40 can be removably attached to the main body 14 and may contain at least two cartridge retainers 50 and 52. Each retainer is configured so as to contain one medicament reservoir, such as a glass cartridge. Preferably, each cartridge contains a different medicament.

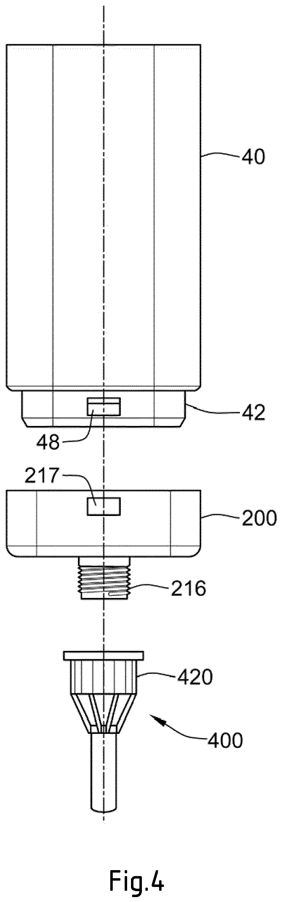

In addition, at the distal end of the cartridge holder 40, the drug delivery device illustrated in Fig. 1 includes a dispense interface 200. As will be described in relation to Fig. 4, in one arrangement, this dispense interface 200 includes a main outer body 212 that is removably attached to a distal end 42 of the cartridge housing 40. As can be seen in Fig. 1, a distal end 214 of the dispense interface 200 preferably comprises a needle hub 216. This needle hub 216 may be configured so as to allow a dose dispenser, such as a conventional pen type injection needle assembly, to be removably mounted to the drug delivery device 10.

Once the device is turned on, the digital display 80 shown in Fig. 1 illuminates and provides the user certain device information, preferably information relating to the medicaments contained within the cartridge holder 40. For example, the user is provided with certain information relating to both the primary medicament (Drug A) and the secondary medicament (Drug B).

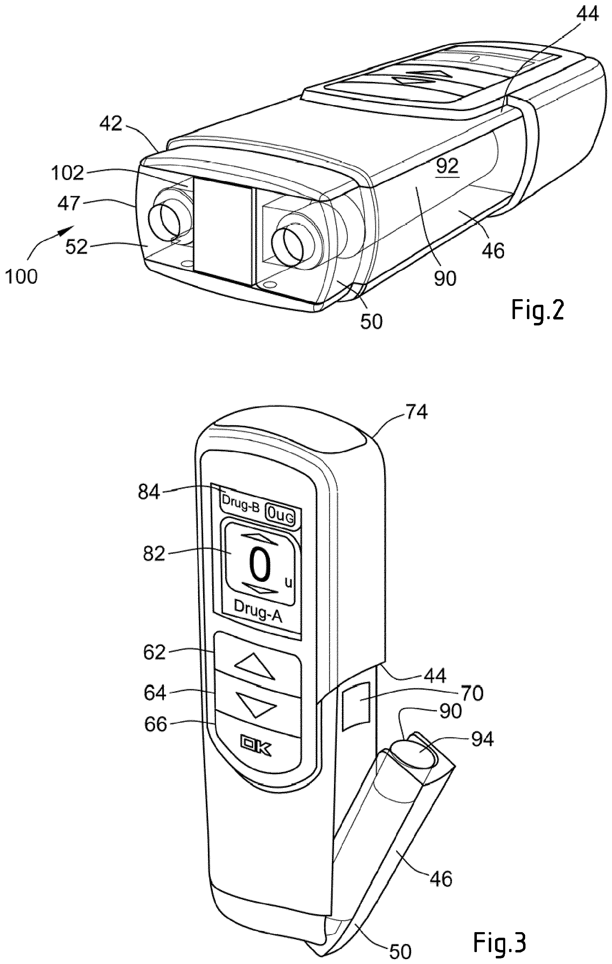

As shown in Fig. 3, the first and second cartridge retainers 50, 52 may be hinged cartridge retainers. These hinged retainers allow user access to the cartridges. Fig. 3 illustrates a perspective view of the cartridge holder 40 illustrated in Fig. 1 with the first hinged cartridge retainer 50 in an open position. Fig. 3 illustrates how a user might access the first cartridge 90 by opening up the first retainer 50 and thereby having access to the first cartridge 90.

As mentioned above when discussing Fig. 1, a dispense interface 200 can be coupled to the distal end of the cartridge holder 40. Fig. 4 illustrates a flat view of the dispense interface 200 unconnected to the distal end of the cartridge holder 40. A dose dispenser or needle assembly 400 that may be used with the interface 200 is also illustrated and is provided in a protective outer cap 420.

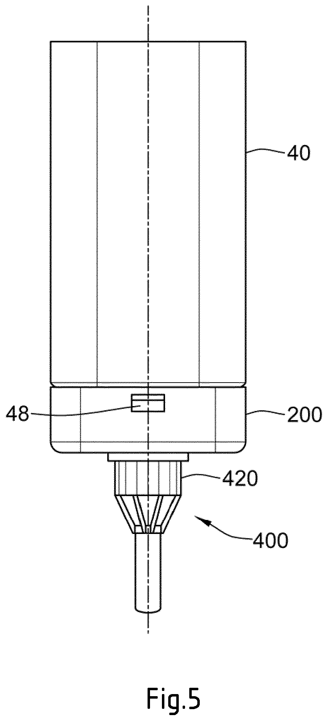

In Fig. 5, the dispense interface 200 illustrated in Fig. 4 is shown coupled to the cartridge holder 40. The axial attachment means 48 between the dispense interface 200 and the cartridge holder 40 can be any known axial attachment means to those skilled in the art, including snap locks, snap fits, snap rings, keyed slots, and combinations of such connections. The connection or attachment between the dispense interface and the cartridge holder may also contain additional features (not shown), such as connectors, stops, splines, ribs, grooves, pips, clips and the like design features, that ensure that specific hubs are attachable only to matching drug delivery devices. Such additional features would prevent the insertion of a non-appropriate secondary cartridge to a non-matching injection device.

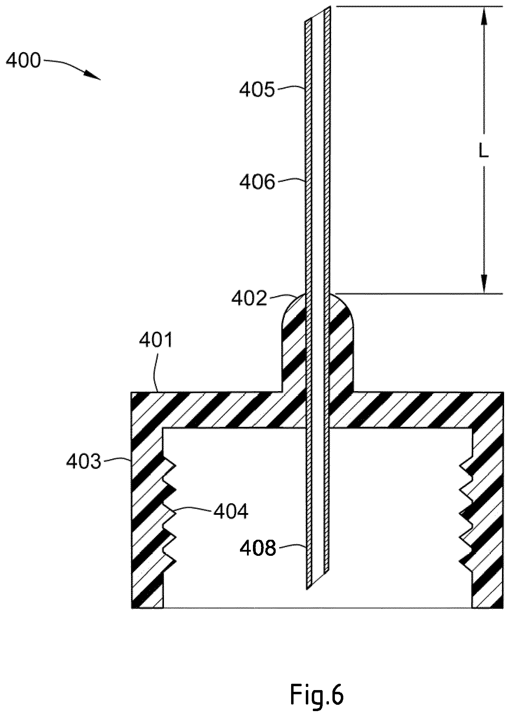

Fig. 5 also illustrates the needle assembly 400 and protective cover 420 coupled to the distal end of the dispense interface 200 that may be screwed onto the needle hub of the interface 200. Fig. 6 illustrates a cross sectional view of the double ended needle assembly 400 mounted on the dispense interface 200 in Fig. 5.

The needle assembly 400 illustrated in Fig. 6 comprises a double ended needle 406 and a hub 401. The double ended needle or cannula 406 is fixedly mounted in a needle hub 401. This needle hub 401 comprises a circular disk shaped element which has along its periphery a circumferential depending sleeve 403. Along an inner wall of this hub member 401, a thread 404 is provided. This thread 404 allows the needle hub 401 to be screwed onto the dispense interface 200 which, in one preferred arrangement, is provided with a corresponding outer thread along a distal hub. At a center portion of the hub element 401 there is provided a protrusion 402. This protrusion 402 projects from the hub in an opposite direction of the sleeve member. A double ended needle 406 is mounted centrally through the protrusion 402 and the needle hub 401. This double ended needle 406 is mounted such that a first or distal piercing end 405 of the double ended needle forms an injecting part for piercing an injection site (e.g., the skin of a user).

Similarly, a second or proximal piercing end 408 of the needle assembly 400 protrudes from an opposite side of the circular disc so that it is concentrically surrounded by the sleeve 403. In one needle assembly arrangement, the second or proximal piercing end 408 may be shorter than the sleeve 403 so that this sleeve to some extent protects the pointed end of the back sleeve. The needle cover cap 420 illustrated in Fig. 4 and 5 provides a form fit around the outer surface 403 of the hub 401.

Referring now to Fig. 4 to 11, one preferred arrangement of this interface 200 will now be discussed. In this one preferred arrangement, this interface 200 comprises:

a. a main outer body 210,
b. an first inner body 220,
c. a second inner body 230,
d. a first piercing needle 240,
e. a second piercing needle 250,
f. a valve seal 260, and
g. a septum 270.

The main outer body 210 comprises a main body proximal end 212 and a main body distal end 214. At the proximal end 212 of the outer body 210, a connecting member is configured so as to allow the dispense interface 200 to be attached to the distal end of the cartridge holder 40. Preferably, the connecting member is configured so as to allow the dispense interface 200 to be removably connected the cartridge holder 40. In one preferred interface arrangement, the proximal end of the interface 200 is configured with an upwardly extending wall 218 having at least one recess.

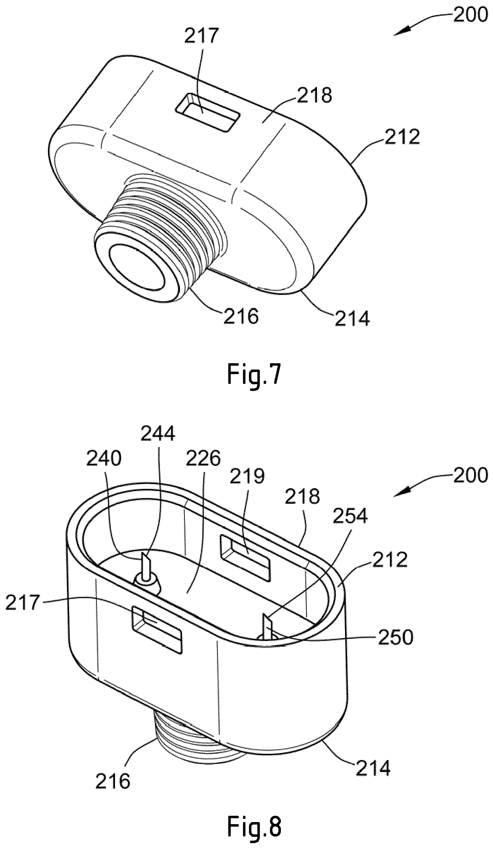

For example, as may be seen from Fig. 8, the upwardly extending wall 218 comprises at least a first recess 217 and a second recess 219.

Preferably, the first and the second recesses 217, 219 are positioned within this main outer body wall so as to cooperate with an outwardly protruding member located near the distal end of the cartridge housing 40 of the drug delivery device 10. For example, this outwardly protruding member 48 of the cartridge housing may be seen in Fig. 4 and 5. A second similar protruding member is provided on the opposite side of the cartridge housing. As such, when the interface 200 is axially slid over the distal end of the cartridge housing 40, the outwardly protruding members will cooperate with the first and second recess 217, 219 to form an interference fit, form fit, or snap lock. Alternatively, and as those of skill in the art will recognize, any other similar connection mechanism that allows for the dispense interface and the cartridge housing 40 to be axially coupled could be used as well.

The main outer body 210 and the distal end of the cartridge holder 40 act to form an axially engaging snap lock or snap fit arrangement that could be axially slid onto the distal end of the cartridge housing. In one alternative arrangement, the dispense interface 200 may be provided with a coding feature so as to prevent inadvertent dispense interface cross use. That is, the inner body of the hub could be geometrically configured so as to prevent an inadvertent cross use of one or more dispense interfaces.

A mounting hub is provided at a distal end of the main outer body 210 of the dispense interface 200. Such a mounting hub can be configured to be releasably connected to a needle assembly. As just one example, this connecting means 216 may comprise an outer thread that engages an inner thread provided along an inner wall surface of a needle hub of a needle assembly, such as the needle assembly 400 illustrated in Fig. 6. Alternative releasable connectors may also be provided such as a snap lock, a snap lock released through threads, a bayonet lock, a form fit, or other similar connection arrangements.

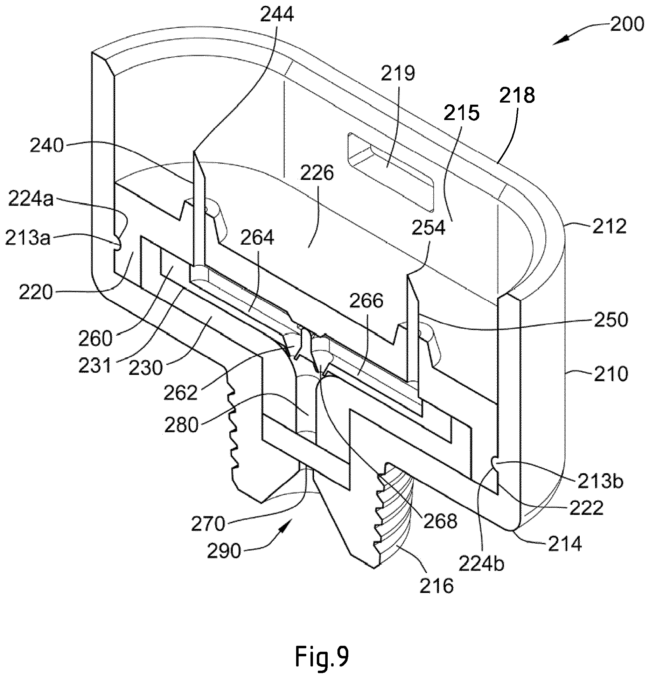

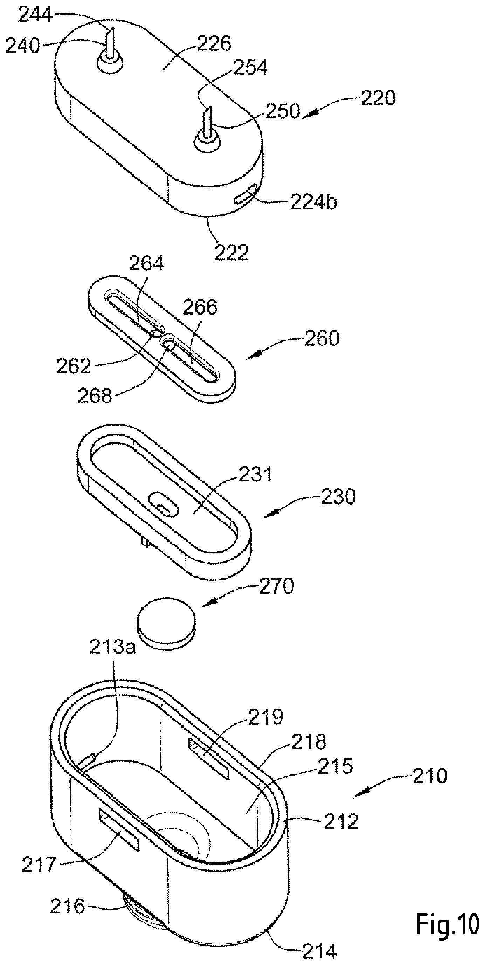

The dispense interface 200 further comprises a first inner body 220. Certain details of this inner body are illustrated in Fig. 8-11. Preferably, this first inner body 220 is coupled to an inner surface 215 of the extending wall 218 of the main outer body 210. More preferably, this first inner body 220 is coupled by way of a rib and groove form fit arrangement to an inner surface of the outer body 210. For example, as can be seen from Fig. 9, the extending wall 218 of the main outer body 210 is provided with a first rib 213a and a second rib 213b. This first rib 213a is also illustrated in Fig. 10. These ribs 213a and 213b are positioned along the inner surface 215 of the wall 218 of the outer body 210 and create a form fit or snap lock engagement with cooperating grooves 224a and 224b of the first inner body 220. In a preferred arrangement, these cooperating grooves 224a and 224b are provided along an outer surface 222 of the first inner body 220.

In addition, as can be seen in Fig. 8-10, a proximal surface 226 near the proximal end of the first inner body 220 may be configured with at least a first proximally positioned piercing needle 240 comprising a proximal piercing end portion 244. Similarly, the first inner body 220 is configured with a second proximally positioned piercing needle 250 comprising a proximally piercing end portion 254. Both the first and second needles 240, 250 are rigidly mounted on the proximal surface 226 of the first inner body 220.

Preferably, this dispense interface 200 further comprises a valve arrangement. Such a valve arrangement could be constructed so as to prevent cross contamination of the first and second medicaments contained in the first and second reservoirs, respectively. A preferred valve arrangement may also be configured so as to prevent back flow and cross contamination of the first and second medicaments.

In one preferred system, dispense interface 200 includes a valve arrangement in the form of a valve seal 260. Such a valve seal 260 may be provided within a cavity 231 defined by the second inner body 230, so as to form a holding chamber 280. Preferably, cavity 231 resides along an upper surface of the second inner body 230. This valve seal comprises an upper surface that defines both a first fluid groove 264 and second fluid groove 266. For example, Fig. 9 illustrates the position of the valve seal 260, seated between the first inner body 220 and the second inner body 230. During an injection step, this seal valve 260 helps to prevent the primary medicament in the first pathway from migrating to the secondary medicament in the second pathway, while also preventing the secondary medicament in the second pathway from migrating to the primary medicament in the first pathway. Preferably, this seal valve 260 comprises a first non-return valve 262 and a second non-return valve 268. As such, the first non-return valve 262 prevents fluid transferring along the first fluid pathway 264, for example a groove in the seal valve 260, from returning back into this pathway 264. Similarly, the second non-return valve 268 prevents fluid transferring along the second fluid pathway 266 from returning back into this pathway 266.

Together, the first and second grooves 264, 266 converge towards the non-return valves 262 and 268 respectively, to then provide for an output fluid path or a holding chamber 280. This holding chamber 280 is defined by an inner chamber defined by a distal end of the second inner body both the first and the second non return valves 262, 268 along with a pierceable septum 270. As illustrated, this pierceable septum 270 is positioned between a distal end portion of the second inner body 230 and an inner surface defined by the needle hub of the main outer body 210.

The holding chamber 280 terminates at an outlet port of the interface 200. This outlet port 290 is preferably centrally located in the needle hub of the interface 200 and assists in maintaining the pierceable seal 270 in a stationary position. As such, when a double ended needle assembly is attached to the needle hub of the interface (such as the double ended needle illustrated in Fig. 6), the output fluid path allows both medicaments to be in fluid communication with the attached needle assembly.

The hub interface 200 further comprises a second inner body 230. As can be seen from Fig. 9, this second inner body 230 has an upper surface that defines a recess, and the valve seal 260 is positioned within this recess. Therefore, when the interface 200 is assembled as shown in Fig. 9, the second inner body 230 will be positioned between a distal end of the outer body 210 and the first inner body 220. Together, second inner body 230 and the main outer body hold the septum 270 in place. The distal end of the inner body 230 may also form a cavity or holding chamber that can be configured to be fluid communication with both the first groove 264 and the second groove 266 of the valve seal.

Axially sliding the main outer body 210 over the distal end of the drug delivery device attaches the dispense interface 200 to the multi-use device. In this manner, a fluid communication may be created between the first needle 240 and the second needle 250 with the primary medicament of the first cartridge and the secondary medicament of the second cartridge, respectively.

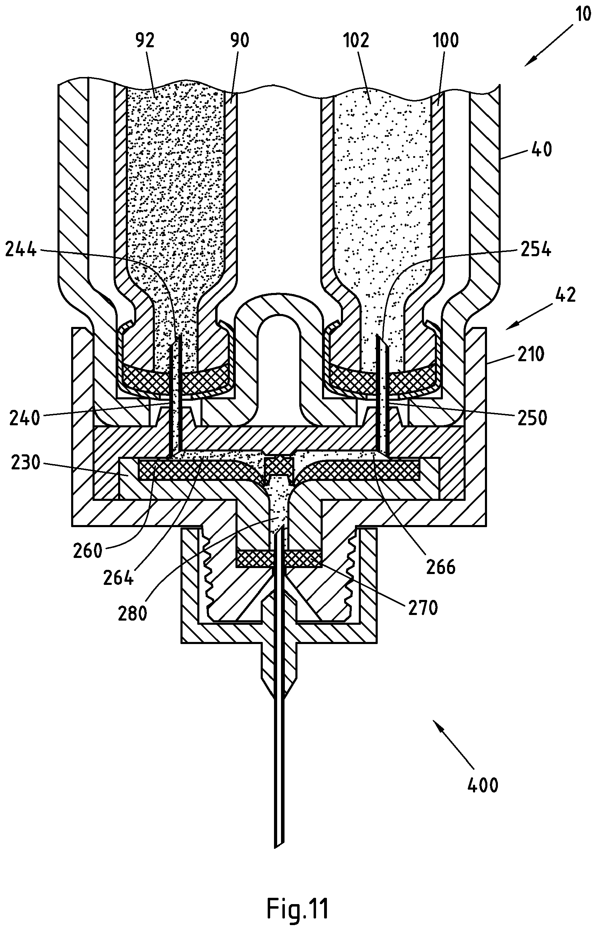

Fig. 11 illustrates the dispense interface 200 after it has been mounted onto the distal end 42 of the cartridge holder 40 of the drug delivery device 10 illustrated in Fig. 1. A double ended needle 400 is also mounted to the distal end of this interface. The cartridge holder 40 is illustrated as having a first cartridge containing a first medicament and a second cartridge containing a second medicament.

When the interface 200 is first mounted over the distal end of the cartridge holder 40, the proximal piercing end 244 of the first piercing needle 240 pierces the septum of the first cartridge 90 and thereby resides in fluid communication with the primary medicament 92 of the first cartridge 90. A distal end of the first piercing needle 240 will also be in fluid communication with a first fluid path groove 264 defined by the valve seal 260.

Similarly, the proximal piercing end 254 of the second piercing needle 250 pierces the septum of the second cartridge 100 and thereby resides in fluid communication with the secondary medicament 102 of the second cartridge 100. A distal end of this second piercing needle 250 will also be in fluid communication with a second fluid path groove 266 defined by the valve seal 260.

Fig. 11 illustrates a preferred arrangement of such a dispense interface 200 that is coupled to a distal end 15 of the main body 14 of drug delivery device 10. Preferably, such a dispense interface 200 is removably coupled to the cartridge holder 40 of the drug delivery device 10.

As illustrated in Fig. 11, the dispense interface 200 is coupled to the distal end of a cartridge housing 40. This cartridge holder 40 is illustrated as containing the first cartridge 90 containing the primary medicament 92 and the second cartridge 100 containing the secondary medicament 102. Once coupled to the cartridge housing 40, the dispense interface 200 essentially provides a mechanism for providing a fluid communication path from the first and second cartridges 90, 100 to the common holding chamber 280. This holding chamber 280 is illustrated as being in fluid communication with a dose dispenser. Here, as illustrated, this dose dispenser comprises the double ended needle assembly 400. As illustrated, the proximal end of the double ended needle assembly is in fluid communication with the chamber 280.

In one preferred arrangement, the dispense interface is configured so that it attaches to the main body in only one orientation, that is it is fitted only one way round. As such as illustrated in Fig. 11, once the dispense interface 200 is attached to the cartridge holder 40, the primary needle 240 can only be used for fluid communication with the primary medicament 92 of the first cartridge 90 and the interface 200 would be prevented from being reattached to the holder 40 so that the primary needle 240 could now be used for fluid communication with the secondary medicament 102 of the second cartridge 100. Such a one way around connecting mechanism may help to reduce potential cross contamination between the two medicaments 92 and 102.

The dispense interface 200 and the needle assembly 400 both form an ejection channel for ejecting a medicament from at least one of the two cartridges 90, 100 out of distal piercing end 405. As illustrated in Fig. 11, the ejection channel is in particular formed by the lumens of the first and second piercing needles 240, 250, of the first and second fluid grooves 264, 266, of the holding chamber 280 and of the double ended needle 406.

The medicaments 92, 102 in cartridges 90, 100 usually contain a preservative, such as m-cresol, for example with a concentration of 2.7 mg/ml. If a medicament dose is ejected from at least one of the cartridges 90, 100 through the ejection channel, the m-cresol concentration in the ejection channel is also 2.7 mg/ml. Over time, however, the m-cresol content in the ejection channel was observed to decrease, so that microbiological integrity of the ejection channel is at risk, in particular if the m-cresol content falls below about 1.8 mg/ml. The m-cresol content may then be too low to ensure a sufficient reduction of bacteria or yeast/fungi.

The m-cresol content level may be restored to a safe level by ejecting a fluid volume from the cartridges 90, 100 through the ejection channel, thereby exchanging at least part of the fluid within the ejection channel. If the device is used on a daily basis, for example by a user injecting insulin at least once per day, a sufficient fluid exchange within the ejection channel is ensured by regular use. However, if the device is not used for a longer time, maybe because a user alternately uses two different devices or forgets to use the device, the m-cresol level in the ejection device may have fallen to a critical level before the device is used for the next time.

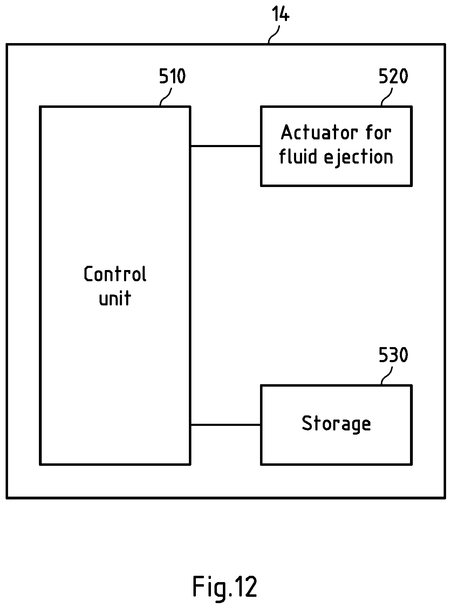

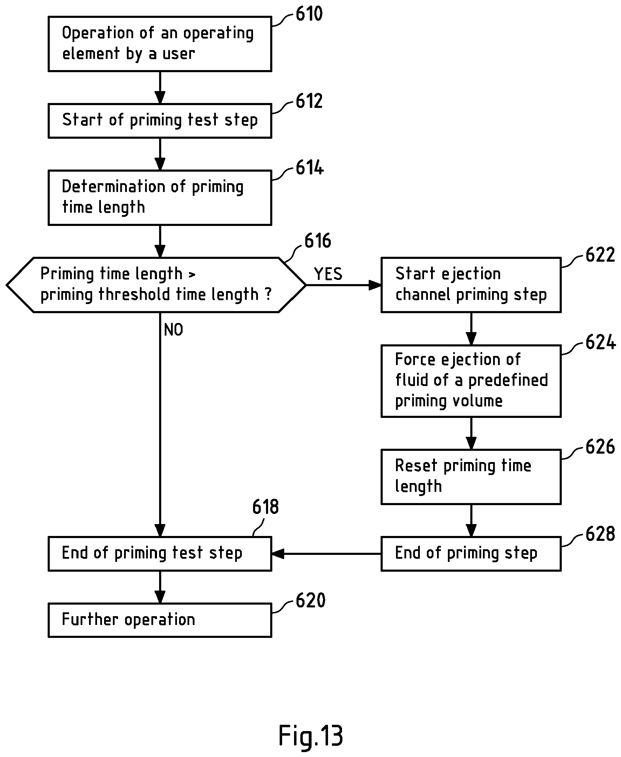

By performing a priming test step, for example once the device is activated by pressing any button of the device or once a user initiates a fluid dose ejection step by pressing injection button 74, microbiological integrity within the ejection channel may be ensured and therewith user safety may be improved. An example for an according control of the delivery device by means of the control unit is now described with reference to Figures 12 and 13.

Figure 12 illustrates a schematic representation of the control unit of the delivery device illustrated in Fig. 1. The main body 14 of the delivery device in particular contains the following components:

> a. a control unit 510;
> b. an actuator for fluid ejection 520; and
> c. a storage 530.

Control unit 510 is a micro-processor control unit and configured to control the functioning of the delivery device. In particular, control unit 510 is configured to control the actuator for fluid injection 520, which may for example be a drive train actuator for actuating a drive train of the device in order to eject a medicament from one of cartridges 90, 100 through the ejection channel. If for example a user presses injection button 74, control unit 510 may perform a fluid dose ejection step, during which control unit 510 controls actuator 520 to actuate the drive train of the device so that a defined medicament dose is ejected from at least one of cartridges 90, 100 through the ejection channel and is, for example, administered to a patient.

The control unit 510 is further configured to determine a priming time length, which is the length of time since the last ejection of a fluid volume through the ejection channel, which fluid volume in the present example is equal to or larger than the volume of the ejection channel of the device. For this purpose, control unit 510 is configured to store the time when a fluid dose ejection step or an ejection channel priming step is performed, so that the priming time length is determinable by subtracting this time from the current time.

Control unit 510 is further configured to perform a priming test step if the user presses injection button 74, which priming test step will now be explained with reference to Figure 13.

Figure 13 shows a flow chart of an exemplary priming test step. Once a user operates an operating element of the device (step 610), in this example injection button 74, control unit 510 starts a priming test step (step 612). In step 614, control unit 510 determines the priming time length $T_{prime}$ by subtracting from the current time $T_{now}$ the time $T_{last\_ejection}$ of the last performance of a fluid dose ejection step or ejection channel priming step, which subtraction corresponds to the following formula:

$$T_{prime} = T_{now} - T_{last\_ejection} \qquad\qquad (1)$$

The value of the priming time length is then compared in step 616 with a predefined priming threshold time length $T_{threshold}$. The priming threshold time length $T_{hreshold}$ may for example be stored in a variable of storage 530, which storage 530 may for example be a RAM, a ROM, a hard drive, a flash memory or the like. For example, the priming threshold time length $T_{threshold}$ may have a value of 48 h.

If the priming time length $T_{prime}$ is smaller than the priming threshold time length $T_{hreshold}$, there is little or no risk for a contamination of the ejection channel since the m-cresol content within the ejection channel should be at a safe level. In this case, control unit 510 therefore ends priming test step (step 618) and continues with controlling further operation of the device (step 620), such as for example performing a fluid dose ejection step.

If however the priming time length $T_{prime}$ exceeds the priming threshold time length $T_{threshold}$, it is likely that the m-cresol level has decreased to a critical level, so that the ejection channel may be contaminated by bacteria, yeast or fungi. In this case, control unit 510 sets the device into a status, in which a further fluid dose ejection step is only allowed after execution of an ejection channel priming step. In this example, this is achieved by control unit 510 immediately starting an ejection channel priming step (step 622), so that this step has to be performed before continuing with further operation of the device.

During the ejection channel priming step, control unit 510 forces ejection of a fluid volume through the ejection channel, which fluid volume is equal to or greater than a predefined minimum priming volume (step 624). The value of the minimum priming volume may for example be stored in a variable in storage 530. In this example, the minimum priming volume is set to the total volume of the ejection channel. To force ejection of the fluid volume, control unit 510 may for example cause displaying of a message such as "Priming required! Please press injection button for priming!" on digital display 80 in order to request initiation of the priming. If the user then presses injection button 74, the control unit 510 controls the actuator for fluid ejection 520 such that a predefined volume is ejected from each one of cartridges 90, 100 through the ejection channel.

Since the m-cresol content in the ejection channel is restored during priming, control unit 510 resets the priming time length $T_{prime}$ (step 626), for example by setting $T_{last\_ejection}$ to $T_{now}$, then ends the ejection channel priming and priming test steps (steps 628, 618) and finally continues with further operation of the device (step 620).

In the course of the invention, a number of experiments were performed to determine, how the microbiological integrity of m-cresol containing medicaments may be preserved. These experiments are described in the following:

First experiment:

In a first experiment, the minimum m-cresol concentration necessary to ensure microbiological integrity was determined.

Test solutions with m-cresol concentrations of between 2.7 mg/ml down to 1.5 mg/ml were inoculated with an inoculum of different bacteria and fungi/yeast and stored for 28 days. During the test, the bacteria and fungi/yeast population of the test solutions were repeatedly analyzed. As an example, the results for the 1.8 mg/ml test solution are presented in table 2.

Table 2: Bacteria/Fungi/Yeast content over time for 1,8mg/ml m-cresol

|  | Bacteria | | | Fungi/Yeast | |
|---|---|---|---|---|---|
|  | Staphilococcus aureus | Escherichia coli | Pseudomonas aeruginosa | Aspergillus niger | Candida albicans |
| Inoculum | 370000 | 330000 | 460000 | 360000 | 340000 |
| Start (T0) | 170000 | 190000 | 23000 | 250000 | 400000 |
| 6 h | 2200 | 55 | <10 |  |  |
| 24 h | <10 | <10 | <10 |  |  |
| 7 d | <10 | <10 | <10 | 15 | 100 |
| 14 d | <10 | <10 | <10 | <10 | 30 |
| 28 d | <10 | <10 | <10 | <10 | <10 |

The analysis presented in table 2 for the 1,8 mg/ml m-cresol test solution was also performed for the test solutions with different m-cresol contents. The results were then compared to the Ph.Eur. and USP criteria to determine, which m-cresol contents are sufficient to fulfil these criteria. The results are summarized in table 3:

Table 3: Comparison of the Ph.Eur. / USP critera fulfilled by the different solutions

| m-cresol content | 1.5 mg/ml | 1.8 mg/ml | 2.1 mg/ml | 2.4 mg/ml | 2.7 mg/ml |
|---|---|---|---|---|---|
| Ph.Eur. | Criterion B OK * | Criterion A OK ** | Criterion A OK | Criterion A OK | Criterion A OK |
| USP | OK | OK | OK | OK | OK |
| * Staph. aur. values after 6 and 24 h fail to fulfil criterion A<br>** Staph. aur. values after 6 h is borderline to criterion B | | | | | |

Table 3 shows that the USP criteria are fulfilled for all m-cresol contents down to 1.5 mg/ml. The Ph.Eur. criterion A is at least fulfilled for all m-cresol contents down to 2.1 mg/ml or even down to 1.8 mg/ml.

It was therefore concluded from this experiment that an m-cresol content of 2.1 mg/ml or even 1.8 mg/ml is still enough to fulfil both the Ph.Eur. criterion A and the USP criteria.

Second experiment:

In a second experiment, the in-use medicament stability was analyzed, i.e. the m-cresol content of a medicament volume in an ejection channel over time.

For the second experiment, a first aqueous test solution comprising

2.7 mg/ml m-cresol,
400 µg/ml lixisenatide (AVE0010), and
100 U/ml insulin glargine (HOE901).

and a second aqueous test solution comprising

2.7 mg/ml m-cresol and
100 U/ml insulin glargine (HOE901)

were provided. In particular, a plurality of cartridges like the cartridge 90 shown in Fig. 2 containing the respective test solutions were provided for both the first and the second test solution.

Furthermore, a plurality of dispense interfaces like the dispense interface 200 shown in Fig. 9 was provided for both the first and the second test solution. Each dispense interface was connected to a needle assembly like the needle assembly 400 shown in Fig. 6. The dead volume of each dispense interface was 20 μl.

The first and second test solutions were each flushed through the respective dispense interfaces, so that each time a volume of 20 μl of the respective solution remained within the respective dispense interface. The dispense interfaces and - as reference - a plurality of filled cartridges for both the first and the second test solution were then stored within an incubator for a maximum time period of 14 days.

At the beginning of the experiment (T0) and after 5 (T5), 10 (T10) and 14 days (T14), one dispense interface and one cartridge at a time were taken out of the incubator for both the first and second test solution, and the m-cresol content of the test solution within the respective dispense interfaces and cartridges were analyzed.

Table 4 summarizes the results of the m-cresol analysis for the test solutions from within the dispense interfaces (Dispense interface) and the cartridges (Reference).

Table 4: m-cresol contents of the first and second test solutions over time

|  | First test solution | | Second test solution | |
| --- | --- | --- | --- | --- |
|  | Dispense interface | Reference | Dispense interface | Reference |
| T0 | 2.7 mg/ml | 2.7 mg/ml | 2.7 mg/ml | 2.7 mg/ml |
| T5 | 2.1 mg/ml | 2.7 mg/ml | 2.1 mg/ml | 2.7 mg/ml |
| T10 | 2.05 mg/ml | 2.7 mg/ml | 1.8 mg/ml | 2.7 mg/ml |
| T14 | 2.0 mg/ml | 2.7 mg/ml | 1.5 mg/ml | 2.7 mg/ml |

The results for the reference solutions from within the cartridges, i.e. from the solutions which were not in contact with the dispense interface material, show a constant m-cresol level of 2.7 mg/ml. The natural m-cresol decay due to ageing, UV light etc. therewith turned out to be negligible for the maximum test time period of 14 days.

In contrast to that, the results for the test solutions from within the dispense interfaces show a drop in the m-cresol level for both the first and second test solution. This decrease in the m-cresol level may therefore be attributed to the contact of the solution with the dispense interface material.

After a time of between 5 and 10 days within the incubator, the m-cresol content of the test solutions within the dispense interfaces dropped below a content of 2.1 mg/ml which according to the first experiment is assumed to be still a safe level for microbiological integrity. Therefore, the ejection channel of a device should be flushed with fresh fluid after a maximum time of between 5 to 10 days to raise again the m-cresol level. Thus, the priming threshold time length is preferably selected from the range of between 24 h to 10 d, preferably from 36 h to 5 d.

The term "drug" or "medicament", as used herein, means a pharmaceutical formulation containing at least one pharmaceutically active compound,

wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,

wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,

wherein in a further embodiment the pharmaceutically active compound comprises at least one peptide for the treatment and/or prophylaxis of diabetes mellitus or complications associated with diabetes mellitus such as diabetic retinopathy,

wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl-ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin.

Exendin-4 for example means Exendin-4(1-39), a peptide of the sequence H His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:

H-(Lys)4-des Pro36, des Pro37 Exendin-4(1-39)-NH2,
H-(Lys)5-des Pro36, des Pro37 Exendin-4(1-39)-NH2,
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;

or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,

H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;

or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (~150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by $\alpha$, $\delta$, $\epsilon$, $\gamma$, and $\mu$. The type of heavy chain present defines the isotype of antibody; these chains are found in IgA, IgD, IgE, IgG, and IgM antibodies, respectively.

Distinct heavy chains differ in size and composition; $\alpha$ and $\gamma$ contain approximately 450 amino acids and $\delta$ approximately 500 amino acids, while $\mu$ and $\epsilon$ have approximately 550 amino acids. Each heavy chain has two regions, the constant region (CH) and the variable region (VH). In one species, the constant region is essentially identical in all antibodies of the same isotype, but differs in antibodies of different isotypes. Heavy chains $\gamma$, $\alpha$ and $\delta$ have a constant region composed of three tandem Ig domains, and a hinge region for added flexibility; heavy chains $\mu$ and $\epsilon$ have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by $\lambda$ and $\kappa$. A light chain has two successive domains: one constant domain (CL) and one variable domain (VL). The approximate length of a light chain is 211 to 217 amino acids. Each antibody contains two light chains that are always identical; only one type of light chain, $\kappa$ or $\lambda$, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are

e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

**Claims**

1.  An apparatus for ejecting a fluid,

    - having a control unit (510) for controlling the apparatus, the control unit (510) being configured to control fluid dose ejection steps, during which a fluid dose is ejected from a fluid reservoir through an ejection channel,
    - wherein the control unit (510) is further configured to determine a priming time length ($T_{prime}$), the priming time length ($T_{prime}$) being the length of time since the last ejection of a fluid volume through the ejection channel,
    - wherein the control unit (510) is further configured to perform a priming test step (612 - 618) comprising the steps of:

        - determining whether the priming time length ($T_{prime}$) is longer than a predefined priming threshold time length ($T_{threshold}$), and
        - if the priming time length ($T_{prime}$) is longer than the priming threshold time length ($T_{threshold}$), setting the apparatus into a status in which a further fluid dose ejection step is only allowed after execution of an ejection channel priming step (622 - 628).

2.  The apparatus according to claim 1, wherein the priming time length ($T_{prime}$) is the length of time since the last ejection of a fluid volume through the ejection channel, which fluid volume is equal to or larger than a predefined minimum ejection volume, in particular equal to or larger than the ejection channel volume.

3.  The apparatus according to claim 1 or 2, wherein the control unit (510) is configured to determine the priming time length ($T_{prime}$) by determining the time length since the last performance of a step from a group of steps, the group of steps at least comprising a fluid dose ejection step and/or an ejection channel priming step (622 - 628).

4.  The apparatus according to any one of claims 1 to 3, wherein the ejection channel priming step (622 - 628) comprises ejection of a fluid volume through the ejection channel, which fluid volume is equal to or larger than a predefined minimum priming volume.

5.  The apparatus according to one of claims 1 to 4, wherein the apparatus further comprises a cartridge retainer (50, 52) configured to hold a fluid containing cartridge (90, 100).

6.  The apparatus according to one of claims 1 to 5, wherein the apparatus further comprises a connector for removably attaching thereto a disposable assembly, the disposable assembly forming at least part of the ejection channel when attached to the connector.

7.  The apparatus according to claim 6, wherein the disposable assembly is a dispense interface (200).

8.  The apparatus according to any one of claims 1 to 7, wherein the apparatus comprises an operating element for operation of the apparatus by a user and wherein the control unit (510) is configured to perform the priming test step (612 - 618) once a user operates the operating element.

9.  The apparatus according to claim 8, wherein the operating element is an operating element for activating the apparatus or for preparing or initiating a fluid dose ejection step.

10. The apparatus according to any one of claims 1 to 9,
wherein the apparatus comprises a housing with a cap connection part for attaching thereto a cap (18) and a sensor configured to determine whether the cap (18) is attached to the connection part, and wherein the control unit (510) is configured to perform the priming test step (612 - 618) upon detachment of the cap (18).

11. The apparatus according to any one of claims 1 to 10,
wherein the predefined priming threshold time length ($T_{threshold}$) is in the range of from 24 h to 10 d, preferably of from 36 h to 5 d.

12. The apparatus according to any one of claims 1 to 11,
wherein the apparatus is a medical device, in particular a medicament injection device.

13. The apparatus according to any one of claims 1 to 12,
wherein the apparatus is hand-held.

14. System, comprising:

   - an apparatus according to any one of claims 1 to 13,
   - a disposable assembly attached to a connection part of the apparatus, the disposable assembly thereby forming at least part of the ejection channel.


**Patentansprüche**

1. Vorrichtung zum Ausstoßen einer Flüssigkeit,

   - umfassend eine Steuereinheit (510) zum Steuern der Vorrichtung, wobei die Steuereinheit (510) zur Steuerung der Flüssigkeitsdosisausstoßschritte ausgelegt ist, während denen eine Flüssigkeitsdosis aus einem Flüssigkeitsreservoir durch einen Ausstoßkanal ausgestoßen wird,
   - wobei die Steuereinheit (510) ferner zur Bestimmung einer Ansaugzeitdauer ($T_{ansaugen}$) ausgelegt ist, wobei die Ansaugzeitdauer ($T_{ansaugen}$) die Zeitdauer seit des letzten Ausstoßes eines Flüssigkeitsvolumens durch den Ausstoßkanal ist,
   - wobei die Steuereinheit (510) ferner zur Durchführung eines Ansaugtestschritts (612-618) ausgelegt ist, der die folgenden Schritte umfasst:

      - Bestimmen, ob die Ansaugzeitdauer ($T_{ansaugen}$) länger als eine vorgegebene Ansaugschwellenzeitdauer ($T_{Schwelle}$) ist, und
      - wenn die Ansaugzeitdauer ($T_{ansaugen}$) länger als die Ansaugschwellenzeitdauer ($T_{Schwelle}$) ist, Einstellen der Vorrichtung in einen Status, in dem ein weiterer Flüssigkeitsdosisausstoßschritt erst nach Durchführung eines Ausstoßkanalansaugschritts (622-628) erlaubt ist.

2. Vorrichtung nach Anspruch 1,
wobei die Ansaugzeitdauer ($T_{ansaugen}$) die Zeitdauer seit des letzten Ausstoßes eines Flüssigkeitsvolumens durch den Ausstoßkanal ist, wobei das Flüssigkeitsvolumen gleich oder größer als ein vorgegebenes Mindestausstoßvolumen ist, insbesondere gleich oder größer als das Ausstoßkanalvolumen ist.

3. Vorrichtung nach Anspruch 1 oder 2,
wobei die Steuereinheit (510) zur Bestimmung der Ansaugzeitdauer ($T_{ansaugen}$) durch Bestimmung der Zeitdauer seit der letzten Durchführung eines Schritts aus einer Gruppe von Schritten ist, wobei die Gruppe von Schritten zumindest einen Flüssigkeitsdosisausstoßschritt und/oder einen Ausstoßkanalansaugschritt (622-628) umfasst.

4. Vorrichtung nach einem der Ansprüche 1 bis 3,
wobei der Ausstoßkanalansaugschritt (622-628) das Ausstoßen eines Flüssigkeitsvolumens durch den Ausstoßkanal umfasst, wobei das Flüssigkeitsvolumen gleich oder größer als ein vorgegebenes Mindestansaugvolumen ist.

5. Vorrichtung nach einem der Ansprüche 1 bis 4,
wobei die Vorrichtung ferner einen Kartuschenhalter (50, 52) umfasst, der zum Halten einer eine Flüssigkeit enthaltenden Kartusche (90, 100) ausgelegt ist.

**6.** Vorrichtung nach einem der Ansprüche 1 bis 5,
wobei die Vorrichtung ferner ein Verbindungsstück zur lösbaren Befestigung einer Einweganordnung daran umfasst,
wobei die Einweganordnung zumindest einen Teil des Ausstoßkanals bildet, wenn sie am Verbindungsstück befestigt ist.

**7.** Vorrichtung nach Anspruch 6,
wobei die Einweganordnung eine Abgabeschnittstelle (200) ist.

**8.** Vorrichtung nach einem der Ansprüche 1 bis 7,
wobei die Vorrichtung ein Bedienungselement zur Bedienung der Vorrichtung durch einen Benutzer umfasst und wobei die Steuereinheit (510) dazu ausgelegt ist, den Ansaugtestschritt (612-618) durchzuführen, wenn ein Benutzer das Bedienungselement bedient.

**9.** Vorrichtung nach Anspruch 8,
wobei das Bedienungselement ein Bedienungselement zur Aktivierung der Vorrichtung oder zur Vorbereitung oder Einleitung eines Flüssigkeitsdosisausstoßschritts ist.

**10.** Vorrichtung nach einem der Ansprüche 1 bis 9,
wobei die Vorrichtung ein Gehäuse mit einem Kappenverbindungsteil zur Befestigung einer Kappe (18) daran und einen Sensor umfasst, der dazu ausgelegt ist zu bestimmen, ob die Kappe (18) am Verbindungsteil befestigt ist, und wobei die Steuereinheit (510) zur Durchführung des Ansaugtestschritts (612-618) beim Abnehmen der Kappe (18) ausgelegt ist.

**11.** Vorrichtung nach einem der Ansprüche 1 bis 10,
wobei die vorgegebene Ansaugschwellenzeitdauer ($T_{Schwelle}$) im Bereich von 24 Std. bis 10 T, vorzugsweise von 36 Std. bis 5 T liegt.

**12.** Vorrichtung nach einem der Ansprüche 1 bis 11,
wobei die Vorrichtung eine medizinische Vorrichtung, insbesondere eine Medikamenteninjektionsvorrichtung ist.

**13.** Vorrichtung nach einem der Ansprüche 1 bis 12, wobei die Vorrichtung in der Hand gehalten wird.

**14.** System, umfassend:

- eine Vorrichtung nach einem der Ansprüche 1 bis 13,
- eine Einweganordnung, die an einem Verbindungsteil der Vorrichtung befestigt ist, wobei die Einweganordnung dadurch zumindest einen Teil des Ausstoßkanals bildet.

**Revendications**

**1.** Appareil destiné à éjecter un liquide,

- ayant une unité de commande (510) destinée à commander l'appareil, l'unité de commande (510) étant configurée pour commander des étapes d'éjection de dose de liquide, pendant lesquelles une dose de liquide est éjectée depuis un réservoir de liquide par un canal d'éjection,
- dans lequel l'unité de commande (510) est également configurée pour déterminer une durée d'amorçage ($T_{amorçage}$), la durée d'amorçage ($T_{amorçage}$) étant la durée depuis la dernière éjection d'un volume de liquide par le canal d'éjection,
- dans lequel l'unité de commande (510) est également configurée pour effectuer une étape d'essai d'amorçage (612-618) comprenant les étapes consistant à :

- déterminer si la durée d'amorçage ($T_{amorçage}$) est plus longue qu'une durée seuil d'amorçage prédéfinie ($T_{seuil}$), et
- si la durée d'amorçage ($T_{amorçage}$) est plus longue que la durée seuil d'amorçage ($T_{seuil}$), régler l'appareil dans un état dans lequel une étape supplémentaire d'éjection de dose de liquide est autorisée uniquement après l'exécution d'une étape d'amorçage de canal d'éjection (622-628).

2. Appareil selon la revendication 1,
dans lequel la durée d'amorçage ($T_{amorçage}$) est la durée depuis la dernière éjection d'un volume de liquide par le canal d'éjection, lequel volume de liquide est égal ou supérieur à un volume minimal d'éjection prédéfini, en particulier égal ou supérieur au volume de canal d'éjection.

3. Appareil selon la revendication 1 ou 2,
dans lequel l'unité de commande (510) est configurée pour déterminer la durée d'amorçage ($T_{amorçage}$) en déterminant la durée depuis la dernière réalisation d'une étape faisant partie d'un groupe d'étapes, le groupe d'étapes comprenant au moins une étape d'éjection de dose de liquide et/ou une étape d'amorçage de canal d'éjection (622-628).

4. Appareil selon l'une quelconque des revendications 1 à 3,
dans lequel l'étape d'amorçage de canal d'éjection (622-628) comprend l'éjection d'un volume de liquide par le canal d'éjection, lequel volume de liquide est égal ou supérieur à un volume minimal d'amorçage prédéfini.

5. Appareil selon l'une quelconque des revendications 1 à 4,
l'appareil comprenant en outre un dispositif de retenue de cartouche (50, 52) configuré pour maintenir une cartouche contenant un liquide (90, 100).

6. Appareil selon l'une quelconque des revendications 1 à 5,
l'appareil comprenant en outre un raccord pour y attacher de façon amovible un ensemble jetable, l'ensemble jetable formant au moins une partie du canal d'éjection lorsqu'il est attaché au raccord.

7. Appareil selon la revendication 6,
dans lequel l'ensemble jetable est une interface de distribution (200).

8. Appareil selon l'une quelconque des revendications 1 à 7,
l'appareil comprenant un élément de fonctionnement pour le fonctionnement de l'appareil par un utilisateur, et l'unité de commande (510) étant configurée pour effectuer l'étape d'essai d'amorçage (612-618) une fois qu'un utilisateur fait fonctionner l'élément de fonctionnement.

9. Appareil selon la revendication 8,
dans lequel l'élément de fonctionnement est un élément de fonctionnement destiné à activer l'appareil ou à préparer ou initier une étape d'éjection de dose de liquide.

10. Appareil selon l'une quelconque des revendications 1 à 9,
l'appareil comprenant un boîtier avec une partie de raccord de couvercle pour y attacher un couvercle (18) et un capteur configuré pour déterminer si le couvercle (18) est attaché à la partie de raccord, et l'unité de commande (510) étant configurée pour effectuer l'étape d'essai d'amorçage (612-618) lors du détachement du couvercle (18).

11. Appareil selon l'une quelconque des revendications 1 à 10,
dans lequel la durée seuil d'amorçage prédéfinie ($T_{seuil}$) se situe dans la plage de 24 heures à 10 jours, de préférence de 36 heures à 5 jours.

12. Appareil selon l'une quelconque des revendications 1 à 11,
l'appareil étant un dispositif médical, en particulier un dispositif d'injection de médicament.

13. Appareil selon l'une quelconque des revendications 1 à 12,
l'appareil étant portatif.

14. Système, comprenant :

- un appareil selon l'une quelconque des revendications 1 à 13,
- un ensemble jetable attaché à une partie de raccord de l'appareil, l'ensemble jetable formant ainsi au moins une partie du canal d'éjection.

Fig.1

EP 3 046 604 B1

Fig.2

Drug-B 0uG

0 u

Drug-A

Fig.3

40

42

48

217

200

216

420

400

Fig.4

Fig.5

Fig.6

200

217

218

212

216

214

**Fig.7**

244

240

226

219

218

254

212

250

217

216

214

200

**Fig.8**

Fig.9

Fig.10

Fig.11

14

510                    520

| Control unit | | Actuator for fluid ejection |

530

Storage

Fig.12

Fig.13

**EP 3 046 604 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012160157 A1
- WO 2012126745 A2
- WO 9412235 A1
- US 6368314 B1

**Non-patent literature cited in the description**

- Rote Liste. 2008
- Remington's Pharmaceutical Sciences. Mark Publishing Company, 1985